# EUROPÄISCHE PATENTSCHRIFT

(11) **EP 3 273 909 B1**
(45) Veröffentlichungstag und Bekanntmachung des Hinweises auf die Patenterteilung: **06.04.2022**
(21) Anmeldenummer: 16707697.5
(22) Anmeldetag: 26.02.2016
(51) Int. Cl.: A61F 2/14

(54) **ARTIFIZIELLE DESCEMETMEMBRAN**
ARTIFICIAL DESCEMET'S MEMBRANE
MEMBRANE DE DESCEMET ARTIFICIELLE

(30) Priorität: 26.03.2015 DE 102015205534
(43) Veröffentlichungstag der Anmeldung: 31.01.2018
(73) Patentinhaber: Fraunhofer-Gesellschaft zur Förderung der angewandten Forschung e.V., 80686 München (DE)
(72) Erfinder: GUTERMUTH, Angela, 52074 Aachen (DE)
(74) Vertreter: Schrell, Andreas
(86) Internationale Anmeldenummer: PCT/EP2016/054101
(87) Internationale Veröffentlichungsnummer: WO 2016/150652

(56) Entgegenhaltungen:
- EP-A1- 1 835 023
- EP-A1- 1 988 152
- US-A1- 2005 214 259
- US-A1- 2010 215 717

## Beschreibung

Die vorliegende Erfindung betrifft Verfahren und Mittel zur Herstellung von cornealem Endothelgewebe des Säugetierauges und stellt ein strukturiertes artifizielles Konstrukt bereit, welches die Neubildung eines Cornealendothels aus isolierten Zellen außerhalb des Körpers ermöglicht.

Die Transparenz der Hornhaut (Cornea) des Auges ist essentiell für die Sehkraft. Sie erfordert vor allem ein intaktes Endothelgewebe (Cornealendothel). Dieses einschichtige Endothel an der Rückseite der Cornea steht in direktem Kontakt mit einer zellfreien Basalmembran, der sogenannten *Lamina limitans posterior descementi* oder Descemet-Membran, welche dieses von der Hauptschicht der Cornea, dem Stroma, abgrenzt. Die Descemet-Membran besteht in der gesunden Cornea im Wesentlichen aus Collagen Typ VIII und Laminin. Sie weist eine regelmäßige räumliche Struktur auf, die mit der Struktur der Zellen des Endothels korrespondiert. Das Endothel entwässert das Stroma aktiv über die Descemet-Membran hinweg und verhindert dort Wassereinlagerung und Ödembildung und damit die Trübung der Cornea.

Humane corneale Endothelzellen arretieren in der G1-Phase und zeigen *in vivo* nur eingeschränkte Proliferationsfähigkeit. Die Teilungsunfähigkeit hat zur Folge, dass es bei Hornhautverletzung, sei sie durch Entzündungsprozesse oder durch mechanisches Trauma hervorgerufen, aber auch aufgrund natürlicher Alterungsprozesse zu einer unwiderruflichen Zerstörung des Endothels und im schlimmsten Falle zu einem kompletten Sehverlust aufgrund Hornhauttrübung kommen kann. Zwar werden abgestorbene Endothelzellen durch benachbarte ersetzt, die sich vergrößern und in Lücken migrieren, um die Dichtigkeit und damit die Funktion des Endothels weiter zu gewährleisten. Dieser Reparaturmechanismus ist jedoch sehr begrenzt.

Dem Endothelzellverlust in der Cornea kann bisher nur beschränkt durch medizinische Maßnahmen entgegengetreten werden. Eine häufig verwendete Methode ist die Substitution der gesamten erkrankten Hornhaut mit einer Spenderhornhaut, der sogenannten perforierenden Keratoplastik. Es treten aber oft klinische Komplikationen auf, die eine ausbleibende oder verminderte Rehabilitation des Auges bedingen, wie chronisch trockene Augen, Corneavaskularisierung (Gefäßeinsprossung) sowie im schlimmsten Fall die Abstoßung des fremden Spendermaterials.

Neben der Totalsubstitution sind inzwischen Verfahren etabliert, worin, nach lokaler Entfernung ausschließlich des erkrankten Endothels zusammen mit dessen Descemet-Membran (Descemetorhexis) anschließend über einen corneoscleralen-cornealen Tunnel ein lamelläres Transplantat, bestehend aus cornealem Endothel und anhängender Descemet-Membran sowie noch einem geringen Anteil anliegenden posteriorem Stroma, aus einer Spenderhornhaut in die Vorderkammer des Empfängers eingebracht wird. Durch diese spezifische Transplantation werden zwar die üblichen Risiken einer Transplantation und die Nebenwirkungen der Operation gegenüber einem Totaltransplantat reduziert. Dennoch bestehen auch hier weitere technische Probleme, die noch nicht überwunden sind.

Problematisch ist bei all diesen allogenen Transplantationen von cornealem Gewebe der auftretende Endothelzellverlust: Die Präparationstechnik am Spenderauge, die Art und Weise der Lagerung sowie das verwendete Nährmedium, der Transport und schließlich die perioperative Handhabung des Spendergewebes gehen stets mit einem mehr oder weniger starken Endothelzellverlust im Transplantat einher. Neben der immunreaktiven Abstoßung des Fremdgewebes können auch diese Faktoren zu einem primären Transplantatversagen führen, wodurch das transplantierte Endothelgewebe funktionslos wird und keine Rehabilitation eintritt. Aus der EP 1 835 023 A1 sind Zellaggregate, die aus isolierten Endothelzellen der menschlichen Cornea stammen, bekannt. Diese dienen der Herstellung eines Cornealendothel-Implantats.

Die Erfindung hat sich die Aufgabe gestellt, diese Nachteile bei der klinischen Reparatur von Cornealendothelgewebe zu überwinden. Besonders sollen die bekannten Nachteile allogener Transplantate, aber auch besonders die Probleme des Endothelzelluntergangs in einem explantierten Spendergewebe reduziert werden.

Zur Lösung stellt die Erfindung Verfahren und Mittel zur Herstellung eines neuartigen biologischen Endothelimplantats bereit, welches die vorbeschriebenen Nachteile bekannter Transplantate überwindet: Ein *de novo* in der Gewebekultur aus isolierten Zellen herstellbares Cornealendothelgewebe auf einer neuartigen artifiziellen Descemet-Membran.

Die Erfinder fanden überraschend, dass durch ein spezielles artifizielles Konstrukt, welches die in der gesunden Hornhaut vorhandene Descemet-Membran spezifisch nachbildet, isolierte omnipotente oder pluripotente Stammzellen angeregt werden können, ein funktionales corneales Endothelgewebe *de novo* mit hoher Zelldichte auszubilden, das zusammen mit dem erfindungsgemäßen artifiziellen Descemet-Konstrukt selbst in das Auge des Patienten implantiert werden kann, um die Funktion der verlorengegangenen Cornealendothels vollständig zu ersetzen.

Die Erfindung stellt dazu ein artifizielles Descemet-Konstrukt bereit, welches besonders geeignet ist, eine mechanotransduktive Differenzierung biologischer Zellen, besonders pluripotenter oder omnipotente Zellen zu einem Cornealendothelgewebe *in vitro* zu unterstützen oder zu ermöglichen.

Ohne an die Theorie gebunden sein zu wollen, kommt es nach dem Aussäen isolierter Zellen auf das erfindungsgemäße Descemet-Konstrukt zu einer mechanotransduktiven Differenzierung der Zellen und dadurch zu einer Ausbildung eines funktionalen Cornealendothelgewebes. Dabei kann auf autologe Zellen, besonders pluripotente oder omnipotente Zellen des Empfängers selbst zurückgegriffen werden. Somit kann ein artifizielles corneales Implantat geschaffen werden, welches aus eigenen Zellen des Empfängers besteht und wobei durch geeignete Kultivierungsmaßnahmen bei der *in vitro* Kultivierung des Gewebes Zellzahl und Zelldichte so gewählt werden kann, dass ein möglicher peri- oder postoperativer Zelluntergang so kompensiert kann, dass auch nach erfolgter Transplantation die Funktion des transplantierten Endothelgewebes dauerhaft erhalten bleibt und eine vollständige dauerhafte Rehabilitation erreicht werden kann.

Erfindungsgemäß besteht das Descemet-Konstrukt aus einem im Wesentlichen kalottenförmigen Grundkörper, wobei in der konkaven Seite (Höhlung) dieses Grundkörpers auf dessen Oberfläche eine Wabenstruktur ausgebildet ist. Die räumliche Wabenstrukturierung der Oberfläche des Grundkörpers ist mittels geeigneter Mikrostrukturierungsverfahren erzeugbar.

Im Zusammenhang mit dieser Erfindung wir unter "Kalotte" oder "kalottenförmig" nicht ausschließlich ein Kugelschnitt im engeren Sinne verstanden, sondern auch Formen, die im Wesentlichen dieser Form entsprechen, darunter fallen Halbschalen- oder Halbkuppelformen wie Abschnitte von Rotationsellipsoiden, Rotationsparaboloiden sowie Übergangsformen davon. Erfindungsgemäß weißt das artifizielle Descemet-Konstrukt besonders eine Form auf, die der Form der Cornea, worin ein durch das oder mit dem Konstrukt gebildetes Implantat eingesetzt werden soll, entspricht.

Besonders ist die Wabenstruktur als solche aus repetitiven geometrischen Grundelementen oder Waben zusammengesetzt gedacht, die wabenförmig bevorzugt lückenlos aneinander angeordnet sind, wobei jedes gedachte Grundelement eine zentrale Vertiefung und diese zu den Nachbargrundelementen hin begrenzende seitliche Stege aufweist. Bevorzugt beträgt die Steghöhe, und damit die Tiefe der zentralen Vertiefung, 0,3 bis 1 µm. Bevorzugt beträgt die Stegbreite 1 bis 8 µm. Bevorzugt beträgt die Breite der von den Stegen umschlossenen Vertiefung von 10 bis 20 µm.

Bevorzugt weisen die Grundelemente jeweils eine hexagonale Grundform auf und bilden somit eine klassische Wabenstruktur. In alternativen Ausführungen ist die Grundform der repetitiven Grundelemente kreisförmig oder im Wesentlichen kreisförmig. In einer alternativen Variante ist die Grundform im Wesentlichen rechteckig oder quadratisch. In einer alternativen Variante ist die Grundform allgemein vieleckig. Bevorzugt wechseln sich Vielecke unterschiedlicher Anzahl an Ecken, bevorzugt mit fünf bis neun Ecken, ab; besonders bevorzugt sind diese Wabenstrukturen aufgebaut aus Hexagonen und Pentagonen.

In einer besonderen Variante ist die Wabenstruktur streng regelmäßig, das heißt sie ist aus im Wesentlichen gleichförmigen und bevorzugt im Wesentlichen gleichgroßen Grundelementen aufgebaut. In einer alternativen und bevorzugten Variante ist die Wabenstruktur unregelmäßig gestaltet. Das heißt, die gedachten Grundelemente weisen jeweils unterschiedliche Größe und/oder unterschiedliche Einzelform auf, wobei die Größen der Grundelemente in der Wabenstruktur bevorzugt (quasi) stochastisch verteilt sind; die mittlere Größe einer Gruppe von 6 bis etwa 24 aneinander liegenden Grundelementen ist dabei konstant gehalten. Die vorgenannten bevorzugten Grö-ßenbereiche werden dabei nicht überschritten.

Bevorzugt sind die Stege, das heißt Seitenwände der Grundelemente jeweils bevorzugt senkrecht zur Grundebene der strukturierten Oberfläche des Grundkörpers angeordnet. In einer alternativen Variante verlaufen die Seitenwände des Grundelements zu dem Boden seiner Vertiefung hin konisch zusammen. Dazu ist besonders vorgesehen, dass die Dicke der Seitenwände zum Boden der Vertiefung hin zunimmt. Die Vertiefung umschließt somit die Form eines vieleckigen Pyramidenstumpfs, beziehungsweise, bei im Wesentlichen kreisförmigem Querschnitt, diejenige eines Kegelstumpfs.

In einer Variante ist ein umlaufender, von der Halbschale abstehender Rand vorgesehen. Dieser braucht nicht strukturiert zu sein. Er dient dem erleichterten Handling des Descemet-Konstrukts zum einen während der Mikrostrukturierung, zum anderen während des Einsatzes des Descemet-Konstrukts bei der Zellkultivierung. Gegebenenfalls kann der Rand nach Abschluss der Zellkulturphase oder vor dem Einsetzen des Descemet-Konstrukts als Bestandteil eines Implantats abgetrennt werden.

In bevorzugter Ausgestaltung enthält das erfindungsgemäße Descemet-Konstrukt ein biologisches oder biokompatibles Polymer und besteht bevorzugt vollständig daraus. Das biologische oder biokompatible Polymer ist bevorzugt mechanisch elastisch und flexibel. In einer alternativen Variante ist es im Wesentlichen unelastisch aber flexibel.

Für die Auswahl der biologischen oder biokompatiblen Polymeren ist relevant, dass, wenn das Descemet-Konstrukt als Bestandteil eines *in vitro* Implantats in das Auge implantiert ist, es postoperativ die physikalischchemische Funktion einer nativen Descemet-Membran ersetzen kann. Dazu zählt insbesondere die Fähigkeit der Durchlässigkeit (passive Diffusion) von Wasser (Kammerwasser) aus dem Stroma hin zu dem aktiv pumpenden Endothelgewebe. Dabei ist besonders vorgesehen, diese Diffusion durch Wahl der Polarität der Materialkomponenten und/oder durch poröse oder durchbrochene Strukturen (Molekularsieb) zu ermöglichen.

Daneben ist vorgesehen, dass das artifizielle Descemet-Konstrukt nach der Implantation teilweise oder vollständig resorbierbar ist. Bevorzugt verbleibt dabei eine Reststruktur, welche die Funktion der nativen Basalmembran übernimmt, wohingegen unmittelbar nach der Implantation zur insbesondere mechanischen Stabilisierung des Implantats in dem Descemet-Konstrukt vorhandene Materialien resorbierbar sind. Auch ist vorgesehen, dass sich eine Basalmembran aus dem artifiziellen Descemet-Konstrukt postoperativ bildet.

Weiter ist vorgesehen, die mechanischen Eigenschaften des biologischen oder biokompatiblen Polymers so zu wählen, dass die mechanischen Eigenschaften einer nativen Descemet-Membran erreicht werden. Besonders ist eine mechanische Steifigkeit des Descemet-Konstrukts gefordert, um einerseits die mechanotransduktive Ausbildung von Cornealendothelgewebe *de novo* aus isolierten Zellen *in vitro* in der Gewebekultur zu ermöglichen und um andererseits eine ausreichende Stabilität bei Implantation in das Empfängerauge peri- und postoperativ zu gewährleisten. In einer Variante ist vorgesehen, dass sich die mechanischen Eigenschaften des Konstrukts während der *in vitro* Kultivierung und/oder postoperativ verändern. Hierzu wird eine Zusammensetzung aus mehreren Komponenten gewählt, die unterschiedliche Resorption zeigen oder mit den Endothelzellen in Wechselwirkung treten.

Das oder die biokompatiblen Polymere in dem Konstrukt können vollständig oder teilweise synthetischen Ursprungs sein. Sie können in an sich bekannter Weise funktionalisiert und insbesondere auch mit adhäsionsfördernden Proteinen oder Peptiden gekoppelt sein.

Das Polymer enthält bevorzugt ein Collagen oder ein Collagengemisch oder besteht bevorzugt daraus. Bevorzugtes Collagen ist Collagen Typ I und/oder Typ II. Besonders bevorzugt ist Collagen Typ VIII und/oder Typ IV. Alternativ bevorzugt sind Mischungen daraus. Bevorzugt ist zusätzlich Laminin enthalten. Bevorzugt ist zusätzlich Fibronektin enthalten. Biochemisch setzt sich die native Descemet-Membran im Auge hauptsächlich aus Collagen Typ IV und VIII, Fibronektin und Laminin zusammen. Es ist bevorzugt vorgesehen, diese extrazellulären Matrixkomponenten bevorzugt nach standardisierten Verfahren und bevorzugt rekombinant zu produzieren. Es wird Mischungsverhältnis der Komponenten eingestellt, so dass die Porengröße in dem artifiziellen Konstrukt und damit das Diffusionspotential der nativen Descemet-Membran erreicht wird. Vorzugsweise wird ein Collagen oder Collagengemisch eingesetzt, welches eine natürliche Nanostrukturierung aufweist. Diese verstärkt die mechanotransduktive Wirkung der erfindungsgemäßen Mikrostrukturierung des Descemet-Konstrukts und beschleunigt und stabilisiert die Differenzierung der isolierten Zellen zu einem Endothelgewebe.

Zusätzlich oder alternativ enthält das Biomaterial rekombinant hergestellte Proteine, die aus der infantilen oder adulten nativen Descemet-Membran des tierischen oder menschlichen Körpers bekannt sind, oder besteht daraus. Die Proteine können xenogenen oder allogenen Ursprungs sein. Bevorzugt ist rekombinant hergestelltes humanes Collagen oder Collagengemische daraus. Alternativ oder zusätzlich enthält das Descemet-Konstrukt Seidenprotein oder besteht daraus. In einer Variante enthält das Konstrukt biokompatible Polymere nichtbiologischen Ursprungs oder besteht ausschließlich daraus.

In einer alternativen Variante ist der Werkstoff des Descemet-Konstrukts synthetische Polymere und Elastomere wie Silikonelastomer oder PMMA, Polyethylene, Polyamide, PVA sowie funktionalisierte Polymere, Misch- und Co-Polymere. Besonders bevorzugt ist thermosensitives oder thermoresponsives Polymer, insbesondere ein N-Isopropylacrylamid, auch Abwandlungen oder Derivate davon sowie auch N,N-Diethylacrylamid (PDEAAm), N-Vinlycaprolactam (PVCL) und vergleichbare.

Bevorzugt ist zumindest die Oberfläche eines solchen synthetischen Polymers funktionalisiert, beispielsweise durch Beschichtung oder chemische oder physikalische Behandlung des Polymers, um in an sich bekannter Weise eine Kompatibilität zu darauf kultivierten biologischen Zellen zu erzielen. Das Polmer kann durch einen Plasmasterilisationsprozess mit Laminin und/oder Fibronektin oder auch einem anderen Zelladhäsionsprotein über Wasserstoffbrückenbildung verbunden sein. Das Polymer kann durch chemische Kopplung (z.B. EDC/NHS Reaktion) kovalent mit einem Adhäsion fördernden Protein (s.o.) gekoppelt sein.

Beispielsweise wird das Descemet-Konstrukt mittels mikrostrukturierender Verfahren erzeugt. Diese sind bevorzugt ausgewählt aus photolithographischen Strukturierungsverfahren, photolytischer Ablation, mikromechanischer Ablation, sowie Formgießen und Formprägen.

Die Mikrostrukturierung erfolgt dabei bevorzugt durch sogenannte abtragende Verfahren, welche aus einem Werkstoff materialabtragend die gewünschte Wabenstruktur herausarbeiten. Bevorzugt sind Laser-basierte Verfahren (photolytische Ablation). Bei der Laserdirektstrukturierung wird ein fokussierter Laserstrahl auf die Bauteiloberfläche gerichtet, wobei durch Absorption in der randzonennahen Oberfläche die Energie der einfallenden Laserstrahlung in thermische Energie umgewandelt wird. Der Wirkungsgrad dieser Umwandlung wird durch den Absorptionsgrad des Werkstoffs in Abhängigkeit von der genutzten Laserwellenlänge, der Oberflächenrauheit, dem Einstrahlwinkel und der Temperatur bestimmt. Abweichend davon kann die Strahlungsenergie direkt chemische Bindungen aufbrechen. Alternativ oder zusätzlich beinhaltet der Materialabtrag einen Phasenwechsel im Werkstoff von fest zu flüssig und nachfolgend zu gasförmig. In allen Fällen wird im Bereich des fokussierten Laserstrahls Werkstoff in Form von Partikeln und/oder Materialschmelze abgetragen. Bei gepulsten Lasern erzeugt dies insbesondere eine einzelne Abtraggeometrie pro Laserpuls, meist in Form eines Rotationsparaboloids ("Näpfchen").

Die Erzeugung zusammenhängender Strukturen geschieht durch eine geeignete Bewegung des Laserstrahls über die Werkstückoberfläche. Dies wird bevorzugt mit Hilfe so genannter Laserscanner realisiert, die einen oder mehrere bewegliche Spiegel beinhalten, die über schnelle Motoren zugestellt werden. Die Qualität des Abtrags hinsichtlich erreichbarer Strukturgröße, Strukturschärfe und Genauigkeit lassen sich dabei durch geeignete Wahl der Laserquelle wählen. Entscheidend dabei sind die Wellenlänge des Laserlichts, die Ausgangsleistung (im Falle gepulster Laser die Einzelpulsenergie) sowie die Strahlqualität der Quelle.

Bevorzugte alternative Mikrostrukturierungsverfahren sind mechanische spanabtragende Verfahren, besonders das Mikro-Diamantfräsen mit mikrokristallinen Werkzeugen. Strukturgrößen im Submikrometerbereich werden dabei nur durch eine Kombination besonderer Maschinentechnik und hochscharfer Werkzeuge erzeugt. Strukturgrößen in diesem Bereich bedienen sich der sogenannten Ultrapräzisionstechnik. Anwendung finden hierbei bevorzugt monokristalline Diamantwerkzeuge, die durch ihre Härte und Schärfe und durch ihre präzisen Schnitte die Herstellung der gewünschten Strukturen ermöglichen. Die Fertigung von ultrapräzisen Komponenten im Drehprozess ist bevorzugt auf die Herstellung rotationssymmetrischer Oberflächen beschränkt. Durch Erweiterung auf die sogenannte Unrundbearbeitung können alternativ auch Oberflächen und Strukturen erzeugt werden, die nicht symmetrisch zur Rotationsachse sind.

Alternativ bevorzugte Ansätze zur Mikrostrukturierung des Descemet-Konstrukts sind sogenannte generierende Verfahren, wobei sich die erfindungsgemäße Wabenstruktur durch lokalen Materialaufbau beziehungsweise Materialaushärtung an einem Grundkörper durch Materialauftrag bildet. Bevorzugte Verfahren sind die Zwei-Photonen-Lithographie, die Interferenzlithographie und die Graustufenlithographie.

Die Zwei-Photonen-Lithographie (2PL) basiert auf dem physikalischen Effekt der Zwei-Photonen-Absorption (2PA) und ermöglicht die Herstellung dreidimensionaler Strukturen bei einer lateralen Auflösung von 150 nm und einer axialen Auflösung von 400 nm. Der aufgeweitete Strahl eines Femtosekunden-Faserlasers mit einer Wellenlänge von 780 nm wird in ein invertiertes Mikroskopobjektiv eingekoppelt und von diesem in ein flüssiges, photosensitives Polymer fokussiert. Da der Laser fokussiert und gepulst ist, ist die Intensität im Fokus hoch genug, um innerhalb des Fokus Zwei-Photonen-Absorption zu ermöglichen. In dem entsprechenden Bereich wird anstatt infraroter Strahlung ultraviolette Strahlung mit einer Wellenlänge von 390 nm emittiert. Das flüssige Photopolymer als Bestandteil des Grundkörpers des Konstrukts vernetzt nur bei der Belichtung durch die energiereiche UV-Strahlung, so dass in dem Bereich, in welchem Zwei-Photonen-Absorption stattfindet, das flüssige Polymer an dem Grundkörper aushärtet und die Wabenstruktur ausbildet.

Bei der Interferenzlithographie wird die Zwei- oder Mehrstrahlinterferenz ausgenutzt um das entsprechende Interferenzmuster in einer lichtempfindlichen Fläche abzubilden und zu speichern. Dafür wird ein Laserstrahl (Argonionenlaser) aufgeweitet und (z.B. durch einen Strahlteilerwürfel) in zwei Teilstrahlen geteilt. Diese werden mit Spiegeln (Plan- oder Konkavspiegeln) unter einem bestimmten Winkel überlagert, so dass in Abhängigkeit dieses Winkels sowie der verwendeten Wellenlänge ein Streifenmuster mit einer bestimmten Gitterkonstanten entsteht. Durch wiederholte Anwendung, besonders nach Rotation des Grundkörpers, kann eine Wabenstruktur ausgebildet werden.

Die Grautonlithographie basiert auf der Belichtung einer Maske, deren Muster durch die Belichtung in einem photoempfindlichen Polymer gespeichert wird. Dabei werden unterschiedliche Strukturtiefen durch unterschiedliche Grautöne auf der Maske realisiert. Hinter der Maske bildet sich eine Intensitätsverteilung entsprechend der Grautonverteilung auf der Maske aus. Unter Verwendung eines Positivresists ist die Struktur dort am tiefsten, wo die Intensität am höchsten ist. Für Negativresists verhält sich dieser Zusammenhang umgekehrt.

Besonders bevorzugt zur Herstellung eines biologischen oder biokompatiblen und implantierbaren Descemet-Konstrukts sind Präge- beziehungsweise Gießverfahren und Kombinationen davon, wobei zunächst mittels der vorgenannten abtragenden oder generierenden Verfahren in einem bevorzugt polymeren Werkstoff eine Negativform (Matrize) des gewünschten Descemet-Konstrukts erzeugt wird und anschließend das Descemet-Konstrukt selbst aus biologischem oder biokompatiblen Polymer mittels Gießen und/oder Prägen als Positivform an der zuvor hergestellten Matrize erzeugt wird. Durch dieses bevorzugte zweistufige Verfahren, kann eine Mikrostrukturierung auch an biologischen oder biokompatiblen Polymeren erfolgen, die selbst nicht unmittelbar den physikalischen mikrostrukturierenden Verfahren zugänglich wären. Die Abformung ist besonders für alle Biomaterialien vorgesehen, die einem Aushärtungs- beziehungsweise Polymerisationsprozess unterliegen, das heißt von einem flüssigen, gießbaren Aggregatzustand durch Aushärtung einen gelartigen oder festen Zustand gelangen. Zur Abformung härtet es an der Negativform aus (Gießen) oder die Negativform wird in das Material gedrückt (Prägen). Neben reinen Biomaterialien biologischen Ursprungs sind auch die hierin beschriebenen synthetischen Polymere zur Abformung vorgesehen.

Im Folgenden wird ein Verfahren zur Herstellung eines artifiziellen Descemet-Konstrukts beschrieben, das jedoch nicht Teil der Erfindung ist. Das Verfahren weist zumindest die folgenden Schritte auf: : 1.

Bereitstellen eines polymeren Grundkörpers und 2. Ausbilden einer räumlichen Wabenstruktur in einer Oberfläche des Grundkörpers mittels mikrostrukturierendem Verfahren. Das Verfahren ist bevorzugt ausgewählt aus:
photolithographischer Strukturierung, photolytischer Ablation, mikromechanischer Ablation, Formgießen und Formprägen.

Bevorzugt enthält der Schritt der Ausbildung der räumlichen Wabenstruktur die Unterschritte: 2.1. Erstellen einer negativen Form (Matrize) der Wabenstruktur mittels eines mikrostrukturierenden Verfahrens und anschließend 2.2. Ausbilden der finalen Wabenstruktur in einem biokompatiblen oder biologischen Polymer durch Gießen oder Prägen mittels der erstellten negativen Form.

Beispielsweise wird das hierin beschriebene artifizielle Descemet-Konstrukt in einem Ansatz des "tissue engineering" außerhalb des menschlichen oder tierischen Körpers zur Neuherstellung eines Endothelgewebes aus Einzelzellen eingesetzt, beispielsweisedie Verwendung des artifiziellen Descemet-Konstrukts zur mechanotransduktiven Differenzierung von, insbesondere mesenchymalen, Vorläufer- oder Stammzellen zu einem Cornealendothelgewebe *in vitro.*

Die Zellen, woraus erfindungsgemäß das Cornealendothelgewebe *de novo* an dem Descemet-Konstrukt gebildet werden soll, sind bevorzugt eukaryotische Vorläuferzellen, besonders adulte Stammzellen, embryonale Stammzellen sowie induzierte pluripotente Stammzellen. Bevorzugt sind mesenchymale Zellen, besonders bevorzugt mesenchymale Stammzellen. Diese sind bevorzugt aus Gewebe des Empfängers isoliert. Bevorzugt werden dazu adulte Stammzellen minimal invasiv aus autologem Fettgewebe des Patienten entnommen.

Zur *in vitro* Differenzierung werden die Zellen auf die strukturierten Oberfläche des erfindungsgemäßen Descemet-Konstrukts ausgesät und dort an Ort und Stelle kultiviert. Die Zelldichte beim Aussäen beträgt bevorzugt mindestens 5 x 10⁵/cm². Zur Unterstützung der Differenzierung der Zellen *in vitro* können schließlich zusätzlich an sich bekannte chemische Faktoren eingesetzt werden, welche die Mechanotransduktion der Zellen an dem erfindungsgemäß mikrostrukturierten Descemet-Konstrukt unterstützen. Besonders ist aber vorgesehen, dass die Differenzierung der Zellen ausschließlich auf mechanotransduktivem Wege an der erfindungsgemäßen Mikrostrukturierung des artifiziellen Descemet-Konstrukts erfolgt. Die dadurch erhältlichen *de novo* Cornealendothelgewebe sind daher im Wesentlichen unbelastet von chemischen oder anderen physikalischen Differenzierungsfaktoren, was zum einen die Differenzierung stabilisiert, zum anderen auch die Mortalität dieser Zellen oder deren De-Differenzierung auch postoperativ reduziert.

Gegenstand der Erfindung ist damit auch ein Verfahren zur Herstellung eines *de novo* Cornealendothel-Implantats aus isolierten biologischen Zellen, gemäß Anspruch 8.

Es ist also in dieser Variante vorgesehen, das artifizielle Descemet-Konstrukt zusammen mit den daran zu einem Endothel differenzierten Zellen als Implantat zur Reparatur von Endotheldefekten an der Cornea von Patienten einzusetzen. In dieser Variante sind die vorgenannten Biomaterialien als Werkstoff für das Descemet-Konstrukt bevorzugt.

Als weiterer Gegenstand der Erfindung wird das artifizielle Descemet-Konstrukt zusammen mit dem darauf ausgebildeten cornealen Endothelgewebe als *in vitro* Gewebeimplantat bereitgestellt. Dieses ist in das Auge eines Empfängers, Wirbeltierauge, besonders Säugetierauge, besonders menschliches Auge, implantierbar, um dort die Funktion eines zerstörten cornealen Endothelgewebes zu ersetzen. Gegenstand der Erfindung ist daher auch ein *in vitro* Implantat, enthaltend aus isolierten Zellen *de novo* aufgebautes Cornealendothelgewebe und das artifizielle Descemet-Konstrukt. Das ausdifferenzierte *de novo* Cornealendothelgewebe, welches an das erfindungsgemäße Descemet-Konstrukt adhäriert, kann als konfektioniertes *in vitro* Implantat mittels an sich bekannter Operationstechniken, insbesondere der DMEK (Descemet-Membran endotheliale Keratoplastie) oder DMAEK (Descemet-Membran automatisierte endotheliale Keratoplastie), in das Empfängerauge verbracht werden.

Ferner kann das Verfahren zur Herstellung eines *de novo* Cornealendothel-Implantats aus isolierten biologischen den folgenden Schritt enthalten: Ablösen des Cornealendothelgewebes von dem Descemet-Konstrukt und Erhalt des isolierten Cornealendothelgewebes als das *in vitro* Cornealendothel-Implantat.

Es ist in dieser alternativen Variante also vorgesehen, das nach Ausdifferenzierung der darauf ausgesäten Zellen zu einem Endothelgewebe von dort wieder abzulösen und das isolierte Endothelgewebe als Implantat zur Reparatur von Endotheldefekten an der Cornea von Patienten einzusetzen.

Das Ablösen des Endothelgewebes von dem artifiziellen Descemet-Konstrukt kann nach an sich aus der *in vitro* Gewebekultur bekannten Verfahren erfolgen. Darunter fällt die Verwendung von lösenden Enzymen, Detergenzien und ähnlichem. Es ist vorgesehen, dass die Zellen mit einem schonenden Enzym wie Akkutase behandelt werden. Dabei muss verhindert werden, dass neben den Adhäsionsproteinen, welche die Anhaftung der Endothelzellen an das Substrat vermitteln, nich auch "tight junctions" zwischen den Endothelzellen selbst zerstört werden, was die funktionelle Integrität des Gewebes beeinträchtigen würde.

Bevorzugt ist, alternativ oder zusätzlich, die Oberfläche Descemet-Konstrukts, zumindest an der Seite des Kontaktes mit den Endothelzellen, spezifisch beschichtet oder funktionalisiert, um die Aubildung von Adhäsionsfaktoren zu vermindern und die Ablösung zu erleichtern.

In einer besonders bevorzugten Variante ist, alternativ oder zusätzlich, das synthetische Polymer des Descemet-Konstrukts ein hierin beschriebenes thermosensitives oder thermoresponsives Polymer, bevorzugt ein N-Isopropylacrylamid. In solchen Werkstoffen kann über die Temperatur die Adhäsion beziehungsweise die Ablösung der Endothelzellen vom Substrat, das heißt dem Descemet-Konstrukt gesteuert werden. Bevorzugt wird zum Ablösen der Endothelzellen das Descemet-Konstrukt mit den Endothelzellen nach Abschluss der Kultivierungs-/Differenzierungsphase auf eine niedere Temperatur von etwa 20 °C oder weniger gebracht. Dadurch erfährt das thermosensitive Substrat eine Zustandsänderung, was zur Ablösung des Endothelgewebes als Ganzes führt oder diese weitgehend unterstützt.

Im Zusammenhang mit der Erfindung wird also das *in vitro* ausgebildete cornealen Endothelgewebe als solches als das *in vitro* Gewebeimplantat bereitgestellt. Dieses ist in das Auge eines Empfängers, Wirbeltierauge, besonders Säugetierauge, besonders menschliches Auge, implantierbar, um dort die Funktion eines zerstörten cornealen Endothelgewebes zu ersetzen. Im Zusammenhang mit der Erfindung steht daher auch ein *in vitro* Implantat, bestehend aus dem aus isolierten Zellen *de novo* aufgebautes Cornealendothelgewebe. Das isolierte *de novo* Cornealendothelgewebe kann mittels an sich bekannter Operationstechniken, insbesondere der DMEK (Descemet-Membran endotheliale Keratoplastie) oder DMAEK (Descemet-Membran automatisierte endotheliale Keratoplastie), in das Empfängerauge verbracht werden.

Die Erfindung wird anhand der nachfolgenden Figuren und den Beispielen zu spezifischen Ausführungsformen näher beschrieben.

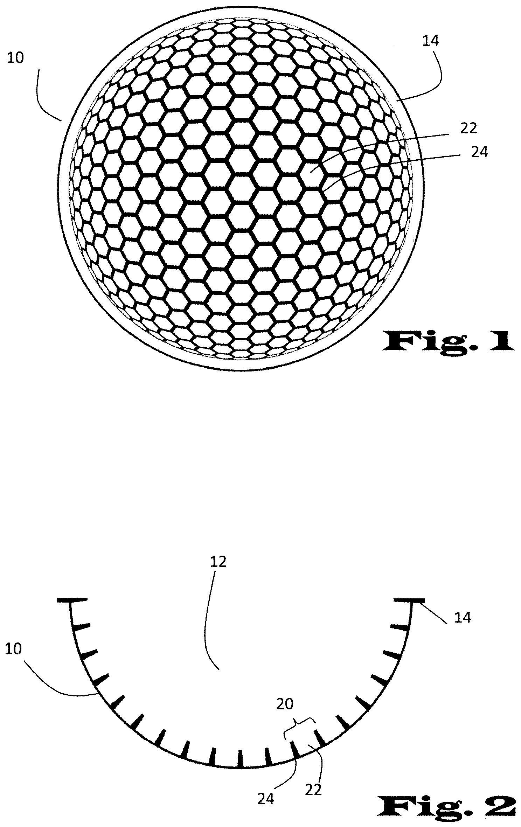

Die Figuren zeigen schematisch und beispielhaft den Aufbau des Descemet-Konstrukts. In den Darstellungen in Figur 1 und Figur 2 sind die Größen nicht skaliert; vielmehr sind die wabenförmigen Strukturen gegenüber der Gesamtgröße des Konstrukts mehrfach vergrößert dargestellt. Figur 1 zeigt eine Draufsicht in die konkave Seite 12 des erfindungsgemäßen kalottenförmigen Descemet-Konstrukts. Figur 2 zeigt eine Querschnittsansicht des Descemet-Konstrukts nach Figur 1. Zur Darstellung der Räumlichkeit der Struktur sind die Wabenstrukturen in Figur 1 zum Rand hin perspektivisch verzerrt. Das Descemet-Konstrukt weist einen Grundkörper 10 in Form einer Kalotte oder Halbschale auf, wobei der entsprechende Kugelradius der Kalotte dem Radius der Cornea im Empfängerauge angepasst ist. Die Wabenstruktur der Mikrostrukturierung ist aus gedachten repetitiven Grundelementen 20 zusammengesetzt, welche jeweils eine zentrale Vertiefung 22 und seitliche als positive Stege 24 ausgebildete Abgrenzungen aufweist. In der dargestellten Ausgestaltung des Descemet-Konstrukts weist dieses randseitig einen umlaufenden überstehenden Rand 14 auf. In der Oberfläche der konkaven Vertiefung 12 des kalottenförmigen Konstrukts 10 ist eine Wabenstruktur aus repetitiven Grundelementen 20, jeweils mit Vertiefungen 22 und seitlichen Stegen 24 vorgesehen. In der dargestellten Ausführung ist an dem oberen Rand des kalottenförmigen Grundkörpers 10 ein umlaufender im Wesentlichen glatter Rand 14 ausgebildet.

### Beispiel 1: Herstellung eines artifiziellen Descemet-Konstrukts

### 1. Lithographische Herstellung einer Matrize

### 1.1 Zwei-Photonen-Lithographie

Die Zwei-Photonen-Lithographie (2PL) basiert auf dem physikalischen Effekt der Zwei-Photonen-Absorption (2PA) und ermöglicht die Herstellung dreidimensionaler Strukturen bei einer lateralen Auflösung von 150 nm und einer axialen Auflösung von 400 nm. Der aufgeweitete Strahl eines Femtosekunden-Faserlasers mit einer Wellenlänge von 780 nm wird in ein invertiertes Mikroskopobjektiv eingekoppelt und von diesem in ein flüssiges, photosensitives Polymer fokussiert. Da der Laser fokussiert und gepulst ist, ist die Intensität im Fokus hoch genug um innerhalb des Fokus Zwei-Photonen-Absorption zu ermöglichen. In dem entsprechenden Bereich wird anstatt infraroter Strahlung ultraviolette Strahlung mit einer Wellenlänge von 390 nm emittiert. Das flüssige Photopolymer vernetzt nur bei der Belichtung durch die energiereiche UV-Strahlung, so dass in dem Bereich, in welchem Zwei-Photonen-Absorption stattfindet, das flüssige Polymer aushärtet.

Durch Bewegung des photosensitiven Polymers relativ zum Fokus können beliebige Strukturen aus Polymer, eingesetzt werden IP-Dip, IP-L, IP-G, OrmoComp, AR-P3120, SU8, hergestellt werden. Ein abschließender Entwicklungsschritt in einem Lösemittel löst das unbelichtete Material von dem belichteten um die Struktur frei zu legen.

Es gibt vier Variationen der Zwei-Photonen-Lithographie, die jeweils für die Herstellung relevanter Strukturen genutzt werden können:
a) Bei dem herkömmlichen Verfahren wird der Laserstrahl durch ein Glasplättchen hindurch in das photosensitive Polymer fokussiert. Zudem befindet sich zwischen dem Mikroskopobjektiv und dem Glasplättchen Immersionsöl, um möglichst viel Laserlicht in das Polymer einzukoppeln und damit eine maximale Auflösung zu ermöglichen. Der Arbeitsabstand des Objektivs begrenzt bei dieser Variation des Verfahrens die maximale Strukturhöhe. Die Struktur wird von der Oberseite des Substrats aus nach oben hin aufgebaut.
b) Die Dip-in Laserlithographie (DiLL) ermöglicht auch die Verwendung opaker Substrate wie Metall oder Silizium. Hier wird der Laserstrahl auf die Unterseite des Substrates fokussiert, das photosensitive Polymer befindet sich zwischen Mikroskopobjektiv und Substrat und dient gleichzeitig als Immersionsmedium. Die Struktur wird von der Substratunterseite aus nach unten hin aufgebaut. Die maximale Strukturhöhe aufgrund der Piezosteuerung liegt bei 300 µm, durch Verwendung des z-Drives des Mikroskopobjektivs können auch weitaus höhere Strukturen realisiert werden.
c) Die dritte Variation besteht in der Verwendung eines Luftobjektivs. Das Prinzip entspricht dem des herkömmlichen Verfahrens, jedoch ohne Verwendung von Immersionsöl.
d) Durch ein AddOn, welches auf Galvoscannern basiert, kann durch das Schreiben mit mehreren Foki gleichzeitig die Schreibgeschwindigkeit auf bis zu 200 fach erhöht werden.

Die Strukturen, welche mit der 2PL hergestellt werden sollen, müssen zuerst programmiert werden. Hierfür kann entweder die Software Describe (für andere Systeme auch Matlab) verwendet werden, die mit der Programmiersprache GWL arbeitet, oder die entsprechende Struktur kann als stl-Datei programmiert und dann dem Programm Nanoslicer übergeben werden. Da das photosensitive Polymer nicht als Material für die Zelldifferenzierung geeignet ist, wird die invertierte Struktur der Descemet-Membran hergestellt und in ein geeignetes Material abgeformt. Verwendet werden Silikonkautschuk oder humanes oder tierisches Collagen. Entsprechend werden sechseckige Noppen realisiert, die Abweichungen in Durchmesser und Höhe aufweisen und zufällig angeordnet sind.

Die Vorgehensweise zur Herstellung der Strukturen gestaltet sich wie folgt:
- Fixierung des Substrats mit Kleber (Fixogum) im Probenhalter
- Auftropfen des entsprechenden Photopolymers auf die Oberseite (für das herkömmliche Verfahren und das Luftobjektiv) oder die Unterseite (für das DiLL-Verfahren) des Substrats
- Für das herkömmliche Verfahren zudem Aufbringung von Immersionsöl an der Unterseite des Substrats
- Fixierung des Probenhalters mit der Probe in dem 2PL-Gerät
- Starten des Schreibvorgangs durch entsprechende Programmierung der Struktur. Die Probe wird entsprechend der programmierten Struktur relativ zum Fokus des Lasers bewegt.

### 1.2. Interferenzlithographie

Bei der Interferenzlithographie wird die Zwei- oder Mehrstrahlinterferenz ausgenutzt um das entsprechende Interferenzmuster in einer lichtempfindlichen Fläche abzubilden und zu speichern. Dafür wird ein Laserstrahl (Argonionenlaser) aufgeweitet und (z.B. durch einen Strahlteilerwürfel) in zwei Teilstrahlen geteilt. Diese werden mit Spiegeln (Plan- oder Konkavspiegeln) unter einem bestimmten Winkel überlagert, so dass in Abhängigkeit dieses Winkels sowie der verwendeten Wellenlänge ein Streifenmuster mit einer bestimmten Gitterkonstanten entsteht.

Die lichtempfindliche Probe, welche in dem Interferenzmuster positioniert wird, besteht aus einem Substrat (Metall, Silizium, Glas), auf welches mittels Spin Coatings eine glatte Schicht aus Photopolymer aufgeschleudert wird (Schichtdicke, Schleuderprogramm). Hierbei können Positiv- oder Negativlacke verwendet werden, wobei Negativlacke dort vernetzen, wo sie belichtet werden, bei Positivlacken bildet der unbelichtete Anteil des Polymers die Struktur.

Um das streifenförmige Interferenzmuster in der Polymerschicht zu speichern, wird eine Probe (Substrat mit Resistschicht) mit dem Interferenzmuster belichtet. Ein darauf folgender Entwicklungsschritt löst (bei Positivresist) das belichtete Polymer aus der Schicht heraus. Damit mit diesem Verfahren sechseckige Noppen hergestellt werden können, muss dieselbe Probe dreimal mit dem Interferenzmuster belichtet werden, wobei die Probe nach jeder Belichtung um 60° gedreht wird. Nach dem Entwicklungsschritt liegen sechseckige Noppen, bestehend aus Kunststoff vor, die ebenfalls für die Abformung der Descemet-Struktur in ein anderes Material verwendet werden können. Die Vorgehensweise gestaltet sich dabei wie folgt: Beschichten eines Substrats mit Photopolymer, je nach Polymer ggf. Ausbacken der Probe auf einer "Hot Plate"; Positionieren der Probe an der Stelle des Interferenzmusters im optischen Aufbau, Dreimaliges Belichten der Probe mit dem Interferenzmuster, wobei die Probe nach jeder Belichtung um 60° in dieselbe Richtung gedreht wird, und Entwickeln der Probe in dem entsprechenden Entwicklerbad

### 1.3 Grauton-Lithographie

Die Grauton-Lithographie basiert auf der Belichtung einer Maske, deren Muster durch die Belichtung in einem photoempfindlichen Polymer gespeichert wird. Dabei werden unterschiedliche Strukturtiefen durch unterschiedliche Grautöne auf der Maske realisiert. Hinter der Maske bildet sich eine Intensitätsverteilung entsprechend der Grautonverteilung auf der Maske aus. Unter Verwendung eines Positivresists ist die Struktur dort am tiefsten, wo die Intensität am höchsten ist. Für Negativresist-Materialien verhält sich dieser Zusammenhang umgekehrt.

Um mit der Grautonlithographie die invertierte Descemet-Struktur herzustellen, muss eine Maske designt werden, welche die Struktur in Grautönen abbildet. Bei Verwendung eines Positivresist-Materialien sind die Konturen der Sechsecke hell und werden zur Mitte hin dunkler. Wird Negativresist-Material verwendet, so sind die Sechsecke auf der Maske in der Mitte hell und werden nach außen hin dunkler. Um eine flächige Ausleuchtung der Maske zu gewährleisten, wird diese senkrecht zur optischen Achse in einen aufgeweiteten und fokussierten Laserstrahl positioniert. Durch die Fokussierung wird die Maske verkleinert auf der Probe abgebildet, so dass die Maße der Struktur originalgetreu sind. Hier gestaltet sich die Vorgehensweise wie folgt: Beschichten eines Substrats mit Photopolymer, je nach Polymer ggf. Ausbacken der Probe auf einer "Hot Plate", Positionieren der Probe an der Stelle des Interferenzmusters im optischen Aufbau, Belichten der Probe mit einem aufgeweiteten, fokussierten Laserstrahl, der unmittelbar nach der Aufweitung die Grauton-Maske durchleuchtet, und Entwickeln der Probe in dem entsprechenden Entwicklerbad.

### 2. Abformen des finalen biologischen Descemet-Konstrukts

Da für die Zelldifferenzierung die Materialeigenschaften von großer Bedeutung sind, wird die so lithographisch hergestellte Struktur in ein weicheres Material, verwendet werden Silikonkautschuk oder tierisches oder humanes Collagen, abgeformt, so dass in dem Material die nachgebildete Descemet-Struktur vorliegt. Zur Herstellung eines implantierbaren Descemet-Konstrukts aus biokompatiblem Material, verwendet wird eine gießfähige Collagenzusammensetzung, wird zunächst eine strukturierte Matrize aus Kunststoff erzeugt.

### Beispiel 2: Herstellung des in vitro Cornealendothel-Gewebes

Mesenchymale Stammzellen, die zuvor aus dem Fettgewebe entnommen und isoliert wurden, werden auf dem gemäß Beispiel 1 hergestellten artifiziellen Descemet-Konstrukt in einer Zelldichte von mindestens 5 x 10⁵ Zellen pro cm² ausgesät. Zur gleichmäßigen Verteilung und Adhäsion wird das Konstrukt orbital geschüttelt.

Nach vollständiger Adhäsion wird die Membran in einen Bioreaktor eingespannt (37 °C, 5 % CO₂, 100 % rel. Feuchte) und solange kultiviert, bis die Stammzellen vollständig differenziert und die Funktionskraft cornealer Endothelzellen erzielt wurde (testbar durch Endothelzell-spezifische Marker beziehungsweise Funktionstests). Die Kultivierungsdauer richtet sich individuell nach der Differenzierungsgeschwindigkeit der konkreten Stammzellen. Zur Kultivierung wird ein basales Kulturmedium verwendet, das zu 10 % Serum und 1 % Antibiotika enthält.

Nach Abschluss der Kultivierung wird ein ausdifferenziertes vollfunktionsfähiges *de novo* Cornealendothel-Gewebe auf dem Descemet-Konstrukt erhalten. Dieses Gewebe haftet auf dem Descemet-Konstrukt an und bildet zusammen mit dem Descemet-Konstrukt ein *in vitro*-Implantat zur Reparatur eines Cornealendotheldefekts in einem Empfängerauge.

## Patentansprüche

1. Artifizielles Descemet-Konstrukt zur mechanotransduktiven Differenzierung biologischer Zellen zu einem *de novo* Cornealendothel-Gewebe, **dadurch gekennzeichnet, dass** das Konstrukt aus einem kalottenförmigen Grundkörper (10) mit einer in der konkaven Seite (12) des Grundkörpers (10) ausgebildeten Wabenstruktur gebildet ist.

2. Konstrukt nach Anspruch 1, wobei die Wabenstruktur aus repetitiven Grundelementen (20) mit zentraler Vertiefung (22) und seitlichen Stegen (24) gebildet ist und die Steghöhe 0,3 bis 1 µm, die Stegbreite 1 bis 8 µm und die Breite der von den Stegen umschlossenen Vertiefung jeweils von 10 bis 20 µm beträgt.

3. Konstrukt nach Anspruch 2, wobei die Wabenstruktur aus repetitiven Grundelementen (20) von im Wesentlichen gleicher Größe gebildet ist.

4. Konstrukt nach Anspruch 2, wobei die Wabenstruktur aus repetitiven Grundelementen (20) verschiedener Größe gebildet ist für eine quasistochastische Verteilung der Größen der Grundelemente (20) in der Wabenstruktur.

5. Konstrukt nach einem der Ansprüche 2 bis 4, wobei die Grundelemente (20) jeweils eine hexagonale Grundform aufweisen.

6. Konstrukt nach einem der vorstehenden Ansprüche, bestehend aus biologischem oder biokompatiblem Polymer.

7. *In vitro* Implantat, enthaltend das Descemet-Konstrukt nach einem der vorstehenden Ansprüche und *de novo* aus isolierten Zellen gebildetes Cornealendothelgewebe.

8. Verfahren zur Herstellung eines *in vitro* Cornealendothel-Implantats aus isolierten biologischen Zellen *de novo,* enthaltend die Schritte:
- Inkontaktbringen isolierter biologischer Zellen mit dem artifiziellen Descemet-Konstrukt nach einem der Ansprüche 1 bis 6, wobei das Konstrukt aus einem kalottenförmigen Grundkörper mit einer in der konkaven Seite des Grundkörpers ausgebildeten Wabenstruktur gebildet ist.
- Kultivierung der Zellen auf der in der konkaven Seite des Grundkörpers ausgebildeten Wabenstruktur des Descemet-Konstrukts, wobei eine Differenzierung der Zellen in Endothelzellen eines cornealen Endothels erfolgt, und
- Erhalten des zu einem *de novo* Cornealendothel ausdifferenzierten Gewebes auf dem Descemet-Konstrukt als das *in vitro* Cornealendothel -Implantat.

9. Verfahren nach Anspruch 8 mit dem weiteren Schritt:
- Ablösen des zu einem *de novo* Cornealendothel ausdifferenzierten Gewebes von dem Descemet-Konstrukt und Erhalten des isolierten Cornealendothels als das *in vitro* Cornealendothel-Implantat.

10. Verfahren nach Anspruch 8 oder 9, wobei die isolierten biologischen Zellen mesenchymale Stammzellen sind.

## Claims

1. Artificial Descemet construct for mechanotransductive differentiation of biological cells into *de novo* corneal endothelial tissue, **characterized in that** the construct is formed from a dome-shaped base body (10) with a honeycomb structure formed in the concave side (12) of the base body (10).

2. Construct according to claim 1, wherein the honeycomb structure is formed from repetitive base elements (20) with central recess (22) and lateral webs (24), the web height being 0.3 to 1 µm, the web width being 1 to 8 µm, and the width of the central recess enclosed by the webs being in each case from 10 to 20 µm.

3. Construct according to claim 2, wherein the honeycomb structure is formed of repetitive base elements (20) of substantially equal size.

4. Construct according to claim 2, wherein the honeycomb structure is formed from repetitive base elements (20) of different sizes for a quasi-stochastic distribution of the sizes of the base elements (20) in the honeycomb structure.

5. Construct according to any one of claims 2 to 4, wherein the base elements (20) each have a hexagonal base shape.

6. Construct according to any one of the preceding claims consisting of a biological or biocompatible polymer.

7. *In vitro* implant including the Descemet construct of any one of the preceding claims and corneal endothelial tissue formed *de novo* from isolated cells.

8. Process of producing an *in vitro* corneal endothelium implant from isolated biological cells *de novo* comprising the steps of:
- contacting isolated biological cells with the artificial Descemet construct of any one of claims 1 to 6, wherein the construct is formed from a dome-shaped base body with a honeycomb structure formed in the concave side of the base body,
- culturing the cells on the honeycomb structure formed on the concave side of the base body of the Descemet construct, wherein differentiation of the cells into endothelial cells of a corneal endothelium takes place, and
- obtaining the tissue differentiated into a *de novo* corneal endothelium with the Descemet construct as the *in vitro* corneal endothelium implant.

9. Process of claim 8, comprising the further step:
- detaching the tissue differentiated into the *de novo* corneal endothelium from the Descemet construct and obtaining the isolated corneal endothelium as the *in vitro* corneal endothelium implant.

10. Process according to claim 8 or 9, wherein the isolated biological cells are mesenchymal stem cells.

## Revendications

1. Construction artificielle de Descemet pour la différenciation mécanotransductive de cellules biologiques en un tissu endothélial cornéen *de novo,* **caractérisée en ce que** la construction est formée d'un corps de base (10) en forme de calotte avec une structure en nid d'abeilles formée dans le côté concave (12) du corps de base (10).

2. Construction selon la revendication 1, dans laquelle la structure en nid d'abeilles est formée d'éléments de base répétitifs (20) avec un creux central (22) et des nervures latérales (24) et la hauteur des nervures est de 0,3 à 1 µm, la largeur des nervures est de 1 à 8 µm et la largeur du creux entouré par les nervures est respectivement de 10 à 20 µm.

3. Construction selon la revendication 2, dans laquelle la structure en nid d'abeilles est formée d'éléments de base répétitifs (20) de taille sensiblement identique.

4. Construction selon la revendication 2, dans laquelle la structure en nid d'abeilles est formée d'éléments de base répétitifs (20) de tailles différentes pour une distribution quasi-stochastique des tailles des éléments de base (20) dans la structure en nid d'abeilles.

5. Construction selon l'une des revendications 2 à 4, dans laquelle les éléments de base (20) ont chacun une forme de base hexagonale.

6. Construction selon l'une des revendications précédentes, constituée d'un polymère biologique ou biocompatible.

7. Implant *in vitro,* contenant la construction de Descemet selon l'une des revendications précédentes et du tissu endothélial cornéen formé *de novo* à partir de cellules isolées.

8. Procédé de fabrication d'un implant d'endothélium cornéen *in vitro* à partir de cellules biologiques isolées *de novo,* comprenant les étapes suivantes :
- Mise en contact de cellules biologiques isolées avec la construction artificielle de Descemet selon l'une des revendications 1 à 6, dans laquelle la construction étant formée d'un corps de base en forme de calotte avec une structure en nid d'abeilles formée dans le côté concave du corps de base.
- la culture des cellules sur la structure en nid d'abeille de la construction Descemet formée dans la côté concave du corps de base, dans laquelle une différenciation des cellules en cellules endothéliales d'un endothélium cornéen étant effectuée, et
- Obtention du tissu différencié en un endothélium cornéen de novo sur la construction de Descemet en tant qu'implant d'endothélium cornéen in vitro.

9. Procédé selon la revendication 8 avec l'étape supplémentaire :
- Détachement du tissu différencié en un endothélium cornéen *de novo* de la construction de Descemet et obtention de l'endothélium cornéen isolé comme implant d'endothélium cornéen *in vitro.*

10. Procédé selon la revendication 8 ou 9, dans lequel les cellules biologiques isolées sont des cellules souches mésenchymateuses.
